(19) Europäisches Patentamt
European Patent Office
Office européen des brevets

(11) Publication number: **0 410 727 A1**

(12) **EUROPEAN PATENT APPLICATION**

(21) Application number: 90308161.0

(51) Int. Cl.[5]: C07D 499/88

(22) Date of filing: 25.07.90

(30) Priority: 26.07.89 JP 193588/89

(43) Date of publication of application: 30.01.91 Bulletin 91/05

(84) Designated Contracting States: AT BE CH DE DK ES FR GB IT LI LU NL SE

(71) Applicant: SUNTORY LIMITED
1-40, Dojimahama 2-chome, Kita-ku
Osaka-shi, Osaka 530(JP)

(72) Inventor: Ishiguro, Masaji
16-17, Fakui-cho
Takarazuka-shi, Hyogo(JP)
Inventor: Iwata, Hiromitsu
27-7, Nanpeidai 5-chome
Takatsuki-shi, Osaka 569(JP)
Inventor: Nakatsuka, Takashi
405, 5-9, Wakayamadai 1-chome,
Shimamoto-cho
Mishima-gun, Osaka 618(JP)
Inventor: Nakajima, Masashi
1-26, Jishikimachi
Takaoka-shi, Toyama(JP)
Inventor: Yamada, Yasuo
267, Kaihotsuhonmachi
Takaoka-shi, Toyama(JP)
Inventor: Doi, Junichi
264, Kaihotsuhonmachi
Takaoka-shi, Toyama(JP)
Inventor: Fujimaru, Masayoshi
1362, Yamadamura-Wakatsuchi, Nei-gun
Toyama(JP)

(74) Representative: Smart, Peter John et al
W.H. BECK, GREENER & CO 7 Stone
Buildings Lincoln's Innlnn
London WC2A 3SZ(GB)

(54) **Processes for removing allyl groups.**

(57) A process for removing an allyl group from a penem compound of formula (I),

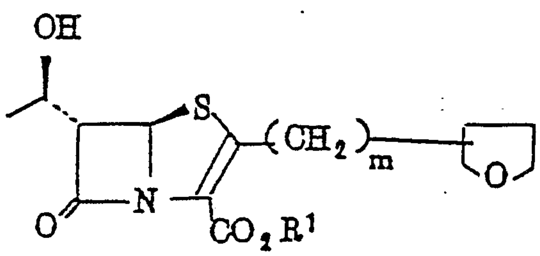

(I)

wherein $R^1$ is an allyl group which may have a substituent and m denotes 0 or 1, is disclosed. The process comprises reacting said penem compound of formula (I) with a cyclic 1,3-diketone compound or its alkali metal enolate.

The carboxylic allyl ester changes to an alkali metal salt of carboxylic acid at the same time the removal of the allyl group from the carboxylic allyl ester occurs, giving a high purity alkali metal salt in a high yield without requiring any additional processes.

EP 0 410 727 A1

## PROCESSES FOR REMOVING ALLYL GROUPS

The present invention relates to a process for removing an allyl group which is very frequently used as a protecting group for carboxyl group or the like in the synthesis of β-lactam antibiotics.

The advantage of the use of allyl group for protecting carboxyl group or the like in the synthesis of β-lactam antibiotics has been reported by S. W. McCombie et al [*J. Org. Chem.*, *47*, 587 (1982)], Japanese Patent Application Laid-open Nos. 94321/1980, 61984/1987, etc. The following processes have been proposed for the removal of the allyl group from the resulting allyl carboxylates.

(i) A process of reacting the allyl carboxylate with an alkali metal carboxylate having 5 to 8 carbon atoms (e.g. sodium 2-ethylhexanoate), as an allyl group acceptor, at room temperature in the presence of tetrakis(triphenylphosphine) palladium(O) and triphenylphosphine as catalysts [*J. Org. Chem.*, *47*, 587 (1982), Japanese Patent Application Laid-open No. 94321/1980].

(ii) A process of reacting the allyl carboxylate with tetrakis(triphenylphosphine) palladium(O) and tributyl-tin-hydride (Japanese Patent Application Laid-open No. 72893/1985).

(iii) A process of reacting the allyl carboxylate with acetyl acetone or dimedone in the presence of tetrakis(triphenylphosphine) palladium(O) (Japanese Patent Application Laid-open No. 61984/1987).

In the above process (i), however, the use of an alkali metal carboxylate as an allyl group acceptor, makes it difficult to separate and purify the target compound from the reaction mixture unless the crystals of the product precipitate in a high yield. The process (ii) is unsuitable or impracticable for large scale production because of a high price of tributyl-tin-hydride and the requirements of conscientious handling of this material, although the process ensures separating the target compound as an alkali carboxylate if a suitable post-treatment is adopted. The process (iii) has a drawback in that it requires additional treatments when the target product is desired in the form of a salt. In addition, in all these known processes, after separation by using a column, the target products are normally obtained by lyophilization or the like means, thus making them unsuitable for industrial application.

In view of this situation, the inventors have conducted extensive studies in order to develop a more advantageous process for the removal of the allyl group from allyl carboxylates which can be applied to industrial production of β-lactam antibiotics. As a result, the inventors have found that when an allyl ester compound of the following formula (I),

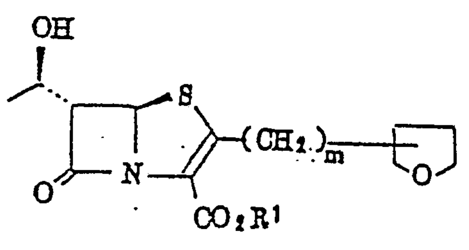

(I)

wherein $R^1$ is an allyl group which may have a substituent and m denotes 0 or 1, was reacted (a) with an alkali metal enolate of 1,3-diketone compound of formula (IIa),

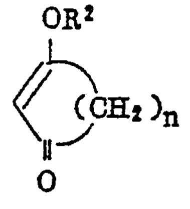

(IIa)

wherein n is an integer of 2 to 4, $R^2$ represents an alkali metal, and the hydrogen atom on the ring may be substituted by a lower alkyl group, a lower alkoxycarbonyl group, a halogen atom, or an aryl group, in the presence of a catalytic amount of a palladium complex, or (b) with a 1,3-diketone compound of formula (IIb),

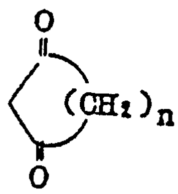

(IIb)

wherein n has the same meaning as defined above and the hydrogen atom on the ring may be substituted by a lower alkyl group, a lower alkoxycarbonyl group, a halogen atom, or an aryl group, in the presence of a salt of an alkali metal, the removal of the allyl group and conversion of the compound of formula (I) into a salt could be performed in one step. This finding has led to the completion of the present invention.

Accordingly, an object of the present invention is to provide a process for removing an allyl group from a penem compound of the following formula (I),

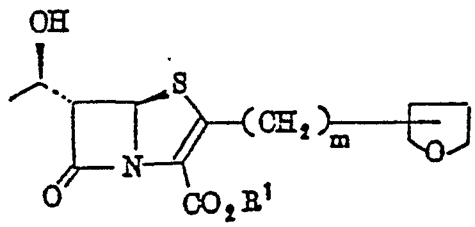

[I]

wherein $R^1$ is an allyl group which may have a substituent and m denotes 0 or 1, which comprises reacting said penem compound of formula (I) (a) with an alkali metal enolate of 1,3-diketone compound of formula (IIa),

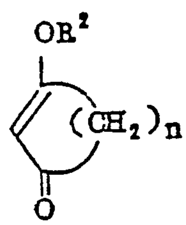

[Ia]

wherein n is an integer of 2 to 4, $R^2$ represents an alkali metal, and the hydrogen atom on the ring may be substituted by a lower alkyl group, a lower alkoxycarbonyl group, a halogen atom, or an aryl group, in the presence of a catalytic amount of a palladium complex, or (b) with a 1,3-diketone compound of formula (IIb),

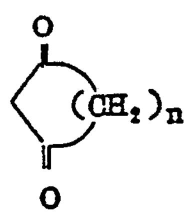

[Ib]

wherein n has the same meaning as defined above and the hydrogen atom on the ring may be substituted by a lower alkyl group, a lower alkoxycarbonyl group, a halogen atom, or an aryl group, in the presence of a salt of an alkali metal, thus converting said compound of formula (I) into an alkali metal salt of penem compound of the following formula (III),

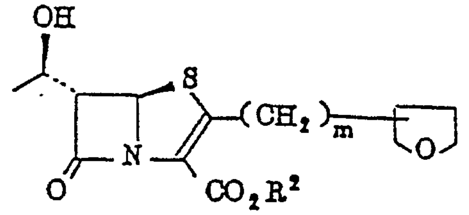

(III)

wherein $R^2$ and m have the same meanings as defined for formula (I).

Another object of the present invention is to provide the hydrate of the above compound of formula (III).

Still another object of the present invention is to provide a process for the separation of the hydrate of the above compound of formula (III) by an addition of water to the mixture obtained by the above reaction.

Other objects, features and advantages of the invention will hereinafter become more readily apparent from the following description.

Examples of allyl groups represented by $R^1$ include allyl alcohol, haloallyl alcohol, methylallyl alcohol, crotyl alcohol, lower alkoxy acid derivatives of these alcohols, cinnamyl alcohol, compounds derived from cinnamyl alcohol active esters, and the like.

A 1,3-diketone compound of formula (IIb) or its alkali metal enolate of formula (IIa) functions as an acyl group acceptor in the reaction of the present invention. The compound of formula (IIb) may be any alicyclic 1,3-diketone compound having a ring with 2 to 4 carbon atoms. The hydrogen atoms on the ring may be substituted by a lower alkyl group, a lower alkoxycarbonyl group, a halogen atom, or an aryl group. Specific examples of such 1,3-diketone compound are 1,3-cyclohexanedione, dimedone, 5,5-dimethyl-4-methoxycarbonyl-1,3-cyclohexanedione and the like. Sodium or potassium enolates are given as an alkali metal enolate of formula (IIa).

Any alkali metal salts which are capable of converting carboxylic acid into alkali carboxylate may be used for the reaction of the compound of formula (I) and the 1,3-diketone compound of formula (IIb). Preferable alkali metal salts are sodium or potassium hydrogencarbonate or carbonate.

The reaction is carried out by the addition of a catalytic amount of a palladium complex to a mixture of the allyl group acceptor and a penem compound of formula (I) which is a substrate compound in a solvent. A preferable proportion of the raw materials is 0.5 to 1.5 equivalent of the allyl group acceptor for one equivalent of the substrate compound.

The reaction proceeds at 10 to 40 ° C with stirring.

As a palladium complex, those having a phosphine ligand is used and particularly triarylphosphine such as triphenylphosphine is preferable as a ligand.

The most preferable palladium complex is tetrakis(triphenylphosphine) palladium(O). Other palladium complexes which can be used include bis(triphenylphosphine) palladium (II) dichloride, dichloro-di-[benzonitrile]palladium (II), and palladium diacetate which are soluble in a solvent and used together with several equivalents of triphenylphosphine (Fieser & Fieser, *Reagent for Organic Synthesis*, Vol. V, 497, 503, 504).

The amount of palladium complex used in the case of tetrakis(triphenylphosphine) palladium(O), for example, is 0.5 to 2.5 mol%. It is possible to shorten the reaction time by using a greater amount of palladium complex. Triphenylphosphine can be used together in order to shorten the reaction time or to decrease the amount of tetrakis(triphenylphosphine) palladium(O).

There are no special restrictions as to the types of the solvent inasmuch as such a solvent dissolves substrate penem compound (I), allyl group acceptor (IIa) or (IIb), and the catalyst palladium complex. Specific examples include haloalkyl compounds, e.g. dichloromethane, chloroform; esters, e.g. methyl acetate, ethyl acetate; ethers, e.g. diethylether, tetrahydrofuran; alcohols, e.g. methanol, ethanol; nitriles, e.g. acetonitrile, propionitrile; ketones, e.g. acetone, methyl ethyl ketone; and aromatic hydrocarbons, e.g. benzene, toluene, xylene. They can be used either independently or, if necessary, as a mixture of two or more.

When the target compound (III) which is produced by the reaction is scarcely soluble in the solvent, the product precipitates as crystals. In this case, the target salt can be collected by filtration. If the target compound is difficult to precipitate from the resulting reaction mixture, the mixture is thoroughly mixed with the solvent used in the reaction and another solvent to which the target compound is scarcely soluble is added to effect the precipitation of the crystals.

The present inventors have discovered that an addition of an appropriate amount of water to the reaction mixture helps precipitation of the target compound as the hydrate which is more crystalline. More precisely, the target compound in the form of hydrate can be obtained by adding water to the reaction

mixture followed by filtration. The target hydrate can also be obtained, instead of filtration, by adding a small amount of inorganic salt such as sodium or potassium hydroxide, sodium or potassium hydrogencarbonate, sodium or potassium carbonate in order to adjust the pH of the mixture, subsequently removing the organic layer and washing the remaining water layer with an organic solvent for several times, then concentrating it. In this method, it is advantageous to add a water-soluble organic solvent, e.g. tetrahydrofuran, acetone, acetonitrile, or the like, at a stage when the concentration of the water layer has proceeded to some extent. This procedure helps to deposit high purity crystals of the target hydrate.

The target crystals thus obtained, sometimes in the form of a hydrate, can be further refined by recrystallization as required.

Other features of the invention will become apparent in the course of the following description of the exemplary embodiments which are given for illustration of the invention and are not intended to be limiting thereof.

## EXAMPLES

### Example 1

To a solution of 16.97 gm (50 mmol) of (1′R,5R,6S)-6-(1′-hydroxyethyl)-2-(3″-tetrahydrofuranyl)-methylpenem-3-carboxylic acid allyl ester, 13.21 gm (60 mmol) of sodium 5,5-dimethyl-4-methoxycarbonyl-1,3-cyclohexanedione enolate, and 0.66 gm (2.5 mmol) of triphenylphosphine in 100 ml of ethyl acetate, 0.58 gm (0.5 mmol) of tetrakis(triphenylphosphine) palladium(O) was added and the mixture was allowed to stand for 2 hours at 25 °C. After the completion of the reaction, 100 ml of water and 10.29 gm of 10% aqueous solution of sodium hydroxide were added to adjust the pH of the solution to 6.5. Then the water layer was separated and washed with 50 ml of methylene chloride followed by concentration of the resulting aqueous solution to 73 gm. To the concentrate 150 ml of acetone was added and the mixture was cooled to 0 °C. The crystals obtained were filtered and dried to obtain 15.57 gm of slightly yellowish crystals of 2.5-hydrate of sodium (1′R,5R,6S)-6-(1′-hydroxyethyl)-2-(3″-tetrahydrofuranyl)methylpenem-3-carboxylate. The purity of the product was 98% by the HPLC analysis (Yield: 83%).

The crystals were dissolved by an addition of 39.06 gm of water and heating. 150 ml of acetone was added to the solution and the mixture was cooled to 0 °C. The crystals obtained were filtered and dried, giving 13.75 gm of white crystals of 2.5-hydrate of sodium (1′R,5R,6S)-6-(1′-hydroxyethyl)-2-(3″-tetrahydrofuranyl)methylpenem-3-carboxylate. The purity of the product was 100% by the HPLC analysis (Yield: 75%).

The properties of the product were as follows:

NMR spectrum: ($D_2O$, $\delta$ ppm)

1.16 (3/2H, d, J=6Hz), 1.17 (3/2H, d, J=6Hz), 1.41-1.62 (1H, m), 1.88-2.04 (1H, m), 2.28-2.46 (1H, m), 2.46-3.00 (1H, m), 3.26-3.40 (1H, m), 3.58-3.82 (4H, m), 4.04-4.17 (1H, m), 5.47 (1H, d, J=2Hz)

IR spectrum: (KBr, $cm^{-1}$) 3400, 1755

$[\alpha]_D^{28}$ : +121° ($H_2O$, c=0.269, converted to dehydrate)

### Example 2

57.7 kg (177 mol) of (1′R,2″R,5R,6S)-6-(1′-hydroxyethyl)-2-(2″-tetrahydrofuranyl)penem-3-carboxylic acid allyl ester was dissolved into 354 l of ethyl acetate. To this solution were added 46.9 kg (213 mol) of sodium 5,5 dimethyl-4-methoxycarbonyl-1,3-cyclohexanedione enolate, 2.3 kg ( 9 mol) of triphenylphosphine, and 2.1 kg (1.8 mol) of tetrakis(triphenylphosphine) palladium(0) and the mixture was allowed to stand for 2 hours at 25 °C. After the completion of the reaction, 355.2 kg of water and 32.7 kg of 10% aqueous solution of sodium hydroxide were added to adjust the pH to 6.5 and to separate the water and organic layers. The water layer was washed with 75 ml of methylene chloride and evaporated under reduced pressure to remove 225 kg of water. To the residue 530 l of acetone was added and the mixture was cooled to 0 °C. The crystals were centrifuged and washed with 15 l of acetone.

The crystals thus obtained were dried, giving 54.1 kg of slightly yellowish crystals of the 2.5-hydrate of sodium (1′R,2″R,5R,6S)-6-(1′-hydroxyethyl)-2-(2″-tetrahydrofuranyl)penem-3-carboxylate. The purity of the compound was 98% by the HPLC analysis (Yield: 85%).

242 kg of water and 5.2 kg of activated charcoal were added to the crystals, and the mixture was

agitated for one hour. The charcoal was removed by a filter. From the filtrate 140 kg of water was removed under reduced pressure. To the residue 410 l of acetone was added and the mixture was cooled to 0 °C. The crystals were centrifuged and washed with 15 l of acetone.

The crystals obtained were dried, giving 48.0 kg of white crystals of 2.5-hydrate of sodium (1′R,2″R,5R,6S)-6-(1′-hydroxyethyl)-2-(2″-tetrahydrofuranyl)penem-3-carboxylate. The purity of the product was 100% by the HPLC analysis (Yield: 77%).

The properties of the product were as follows:

| Elemental analysis: $C_{12}H_{14}NNaO_5S \cdot 2.5H_2O$ | |
|---|---|
| ( Molecular weight 352.343) | |
| Calculated value (C,H,N) :<br>Measured value (C,H,N) : | 40.91, 5.44, 3.98<br>40.78, 5.44, 3.93 |

NMR spectrum: ($CDCl_3$, δ ppm, TMS standard)
1.71 (3H, d, J=6Hz), 1.73-2.03 (3H, m), 2.18-2.35 (1H, m), 3.67-3.92 (3H, m), 4.05-4.19 (1H, m), 5.38 (1H, dd, J=7Hz), 5.46 (1H, s)
IR spectrum: (KBr, $cm^{-1}$) 3280, 1752
$[\alpha]_D^{26}$ : +132.6 ($H_2O$, c=0.99)
X-ray diffraction:
[Measuring Conditions]
Power of X-ray
50 KV, 200 mA
Irradiation source
Cu, graphite monochromater
[Crystal Data]
Size 0.3 X 0.3 X 0.5 mm
Crystalline Differential orthorhombic
Space Group P2, 2, 2

| Lattice Constants | | |
|---|---|---|
| a 9.2078<br>α 90.0 | b 32.4538<br>β 90.0 | c 5.4951<br>γ 90.0 |

Example 3

To 100 ml of tetrahydrofuran were added 16.27 gm (50 mmol) of (1′R,2″R,5R,6S)-6-(1′-hydroxyethyl)-2-(2″-tetrahydrofuranyl)penem-3-carboxylic acid allyl ester, 13.21 gm (60 mmol) of sodium 5,5-dimethyl-4-methoxycarbonyl-1,3-cyclohexane enolate, and 0.66 gm (2.5 mmol) of triphenylphosphine. To this solution 0.58 gm (0.5 mmol) of tetrakis(triphenylphosphine) palladium(O) was added and the mixture was allowed to stand for one hour at 25 °C. After the completion of the reaction, 4.5 gm of water was added and the solution was stirred for 30 minutes.

The crystals were collected by filtration and dried, obtaining 16.74 gm of slightly yellowish crystals of 2.5-hydrate of sodium (1′R,2″R,5R,6S)-6-(1′-hydroxyethyl)-2-(2″-tetrahydrofuranyl)penem-3-carboxylate. The purity of the product was 98% by the HPLC analysis (Yield: 93%).

The crystals were dissolved by an addition of 40.44 gm of water and heating. 150 ml of acetone was added to the solution and the mixture was cooled to 0 °C. The crystals were collected by filtration and dried, obtaining 15.12 gm of white crystals of 2.5-hydrate of sodium (1′R,2″R,5R,6S)-6-(1′-hydroxyethyl)-2-(2″-tetrahydrofuranyl) penem-3-carboxylate. The purity of the product was 100% by the HPLC analysis (Yield: 86%).

Examples 4 - 13

A series of experiments were carried out in a similar manner as in Example 4 varying the reaction solvent and reaction time as shown in Table 1.

The purity and the yield of the products, 2.5-hydrate of sodium (1′R,2″R,5R,6S)-6-(1′-hydroxyethyl)-2-(2″- tetrahydrofuranyl)penem-3-carboxylate, were also shown in Table 1.

Table 1

| Example No. | Solvent | Reaction time (hours) | Purity (%) | Yield (%) |
|---|---|---|---|---|
| 4 | Ethylene glycol dimethyl ether | 1 | 100 | 82 |
| 5 | 1,4-Dioxane | 2 | 100 | 77 |
| 6 | sec-Butanol | 1 | 100 | 76 |
| 7 | Acetonitrile | 4 | 100 | 81 |
| 8 | Acetone | 2 | 100 | 82 |
| 9 | Methyl ethyl ketone | 5 | 100 | 84 |
| 10 | Methyl acetate | 1 | 100 | 82 |
| 11 | 1,2-Dichloroethane | 4 | 99 | 80 |
| 12 | Monochlorobenzene | 2 | 98 | 83 |
| 13 | Toluene | 8 | 98 | 73 |

Example 14

A mixture of (1′R,2″R,5R,6S)-6-(1′-hydroxyethyl)-2-(2″-tetrahydrofuranyl)penem-3-carboxylic acid allyl ester (59.87 gm), 1,3-cyclohexanedione (14.44 gm), tetrakis(triphenylphosphine) palladium(O) (4.25 gm) and tetrahydrofuran (368 ml) was stirred for 13 hours at room temperature. To the reaction mixture were added 170 ml of water and 16 gm of sodium hydrogencarbonate and the mixture was stirred for 30 minutes. Then, the mixture was washed three times with methylene chloride, and 1300 ml of acetone was added to the water layer under ice-cooling with stirring. The mixture was stirred for a further 3 hours at the same temperature, and precipitated crystals were collected by filtration and dried for 2 hours under reduced pressure, obtaining 42.07 gm of white crystals of sodium (1′R,2″R,5R,6S)-6-(1′-hydroxyethyl)-2-(2″-tetrahydrofuranyl)penem-3-carboxylate.

Example 15

A mixture of (1′R,2″R,5R,6S)-6-(1′-hydroxyethyl)-2-(2″-tetrahydrofuranyl)penem-3-carboxylic acid allyl ester (1.952 gm), dimedone (0.474 gm), tetrakis(triphenylphosphine) palladium(O) (69 mg) and tetrahydrofuran (6 ml) was stirred for 3 hours at room temperature. To the reaction mixture were added 7 ml of water and 0.605 gm of sodium hydrogencarbonate and the mixture was stirred for 30 minutes. Then, the mixture was washed three times with methylene chloride, and 35 ml of acetone was added to the water layer under ice-cooling with stirring. The mixture was stirred for a further 3 hours at the same temperature, and precipitated crystals were collected by filtration and dried for 2 hours under reduced pressure, obtaining 1.449 gm of white crystals of sodium (1′R,2″R,5R,6S)-6-(1′-hydroxyethyl)-2-(2″-tetrahydrofuranyl)-penem-3-carboxylate.

Example 16

A mixture of (1′R,2″R,5R,6S)-6-(1′-hydroxyethyl)-2-(2″-tetrahydrofuranyl)penem-3-carboxylic acid allyl ester (325 mg), dimedone (168 mg), tetrakis(triphenylphosphine palladium(O) (12 mg), sodium hydrogencarbonate (92 mg), and tetrahydrofuran (2 ml) was stirred for 3 hours at room temperature. To the reaction mixture was added 2 ml of water and the mixture was stirred for 30 minutes. Then the mixture was washed

three times with methylene chloride, and 10 ml of acetone was added to the water layer under ice-cooling with stirring. The mixture was stirred for a further 3 hours at the same temperature, and precipitated crystals were collected by filtration and dried for 2 hours under reduced pressure, obtaining 253 mg of white crystals of sodium (1′R,2″R,5R,6S)-6-(1′-hydroxyethyl)-2-(2″-tetrahydrofuranyl)penem-3-carboxylate.

Example 17

A mixture of (1′R,2"R,5R,6S)-6-(1′-hydroxyethyl)-2-(2″-tetrahydrofuranyl}penem-3-carboxylic acid allyl ester (325 mg), dimedone (168 mg), tetrakis(triphenylphosphine) palladium(O) (12 mg), potassium hydrogencarbonate (300 mg), and tetrahydrofuran (2 ml) was stirred for 6 hours at room temperature. To the reaction mixture was added 2 ml of water and the mixture was stirred for 30 minutes. Then, the mixture was washed three times with methylene chloride, and 10 ml of acetone was added to the water layer under ice-cooling with stirring. The mixture was stirred for a further 3 hours at the same temperature, and precipitated crystals were collected by filtration and dried for 2 hours under reduced pressure, obtaining 312 mg of white crystals of sodium (1′R,2″R,5R,6S)-6-(1′-hydroxyethyl)-2- (2″-tetrahydrofuranyl)penem-3-carboxylate.

Example 18

To a mixture of dimedone (19 gm) and methanol (50 ml) was added 7.13 gm of sodium methylate under ice-cooling with stirring. The mixture was stirred for 30 minutes at room temperature, and concentrated under reduced pressure. To the residue were added 22.12 gm of (1′R,2″R,5R,6S)-6-(1′-hydroxyethyl)-2-(2″-tetrahydrofuranyl) penem-3-carboxylic acid allyl ester, 763 mg of tetrakis-(triphenylphosphine) palladium(O) and 866 mg of triphenylphosphine and the mixture was stirred for 2.5 hours at room temperature. To this reaction mixture was added 70 ml of water and the pH of the mixture was adjusted to 6.8 by carefully adding 4N sodium hydroxide, and the organic layer was separated. The water layer was washed two times with methylene chloride and 500 ml of acetone was added to the water layer under ice-cooling with stirring. The mixture was stirred for a further 3 hours at the same temperature, and precipitated crystals were collected by filtration and dried for 2 hours under reduced pressure, obtaining 20.43 gm of white crystals of sodium (1′R,2″R,5R,6S)-6-(1′-hydroxyethyl)-2-(2″-tetrahydrofuranyl]-penem-3-carboxylate.

Example 19

To 180 ml of tetrahydrofuran were added 29.28 gm (90 mmol) of (1′R,2″R,5R,6S)-6-(1′-hydroxyethyl)-2-(2″-tetrahydrofuranyl)penem-3-carboxylic acid allyl ester, 13.21 gm (108 mmol) of potassium 5,5-dimethyl-4-methoxycarbonyl- 1,3-cyclohexane enolate, and 1.17 gm (4.5 mmol) of triphenylphosphine. To this solution 2.08 gm (1.8 mmol) of tetrakis(triphenylphosphine) palladium(O) was added and the mixture was allowed to stand for one hour at 25 °C. After the completion of the reaction, 3.1 gm of water was added and the mixture was stirred for 30 minutes.

The crystals thus prepared were collected by filtration and dried, obtaining 31.87 gm of slightly yellowish crystals (crude product) of 2.5-hydrate of potassium (1′R,2″R,5R,6S)-6-(1′-hydroxyethyl)-2-(2″-tetrahydrofuranyl) penem-3-carboxylate.

The crystals were dissolved by an addition of 54 gm of water and heating. 150 ml of tetrahydrofuran was added to the solution and the mixture was cooled to 0 °C. The crystals thus obtained were filtered and dried, obtaining 25.53 gm of white crystals (refined product) of 2.5-hydrate of potassium (1′R,2″R,5R,6S)-6-(1′-hydroxyethyl)-2-(2″-tetrahydrofuranyl)penem-3-carboxylate. The purity of the product was 100% by the HPLC analysis (Yield: 77%).

According to this invention an allyl group can be removed under mild conditions from a penem compound having a carboxylic allyl ester group by using a 1,3-diketone compound or its enolate which are readily available and easy to handle.

In this process, the carboxylic allyl ester changes to an alkali metal salt of carboxylic acid at the same time the removal of tne allyl group from the carboxylic allyl ester occurs. Accordingly, a high purity alkali metal salt can be obtained in a high yield without requiring any additional processes.

The term "lower" used herein in respect of alkyl and alkoxy groups includes $C_1$ to $C_8$ groups, more

preferably $C_1$ to $C_6$ groups, e.g. $C_1$ to $C_3$ groups.

The invention includes a process for removing a protecting allyl group (which may be substituted) from an allyl protected carboxylic acid group comprising reacting the protected group with an alkali metal enolate of a 1,3 diketone of the formula IIa, wherein the hydrogen atoms of the ring may be substituted by one or more substantially non-interfering substituents in the presence of a catalytic amount of a pallidium complex, or with a 1,3 diketone compound of the formula IIb, wherein the hydrogen atoms of the ring may be substituted by one or more substantially non-interfering substituents in the presence of an alkali metal salt and optionally, a catalytic amount of a palladium complex.

## Claims

1. A process for removing an allyl group from a penem compound of the following formula (I),

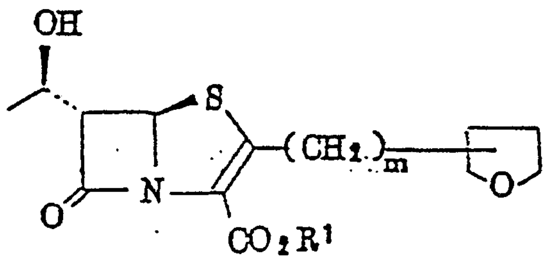

(I)

wherein $R^1$ is an allyl group which may have a substituent and m denotes 0 or 1, which comprises reacting said penem compound of formula (I) (a) with an alkali metal enolate of 1,3-diketone compound of formula (IIa),

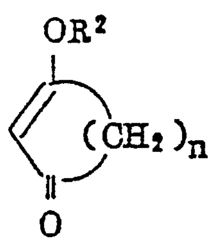

(IIa)

wherein n is an integer of 2 to 4, $R^2$ represents an alkali metal, and the hydrogen atoms on the ring may be substituted by a lower alkyl group, a lower alkoxycarbonyl group, a halogen atom, or an aryl group, in the presence of a catalytic amount of a palladium complex, or (b) with a 1,3-diketone compound of formula (IIb),

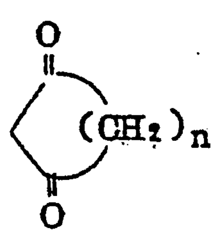

(IIb)

wherein n has the same meaning as defined above and the hydrogen atoms on the ring may be substituted by a lower alkyl group, a lower alkoxycarbonyl group, a halogen atom, or an aryl group, in the presence of a salt of an alkali metal, thus converting said compound of formula (I) into an alkali metal salt of penem compound of the following formula (III),

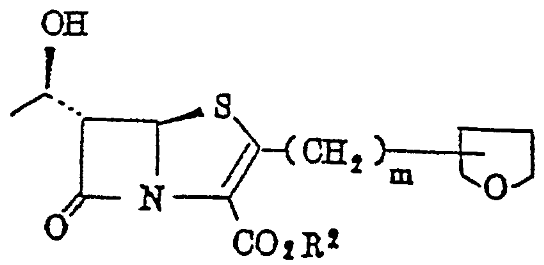

(III)

wherein $R^2$ is an alkali metal and m denotes 0 or 1.

2. A process according to Claim 1, wherein $R^1$ is an allyl, haloallyl, methylallyl, crotyl, or cinnamyl group.

3. A process according to Claim 1, wherein said alkali metal enolate of 1,3-diketone compound is sodium 1,3-cyclohexanedione enolate, sodium dimedone enolate, or sodium 5,5-dimethyl-4-methoxycarbonyl-1,3-cyclohexanediol.

4. A process according to Claim 1, wherein said alkali metal salt is an alkali metal carboxylate.

5. A process according to Claim 4, wherein said alkali metal salt is sodium carboxylate or potassium carboxylate.

6. A process according to Claim 1, wherein said 1,3-diketone compound is 1,3-cyclohexanedione, dimedone, or 5,5-dimethyl-4-methoxycarbonyl-1,3-cyclohexanedione.

7. An alkali metal salt hydrate of penem compound of the following formula (III),

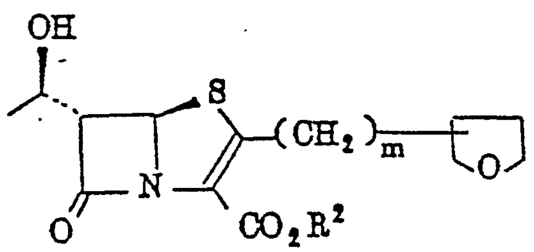

(III)

wherein $R^2$ represents an alkali metal and m denotes 0 or 1.

8. A process for the preparation of an alkali metal salt hydrate of penem compound of the following formula (III),

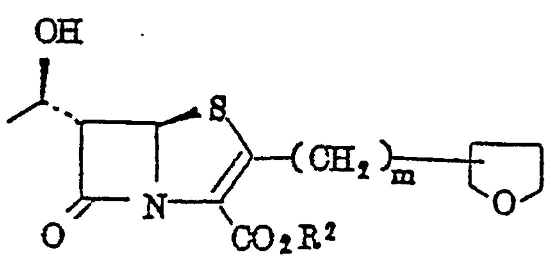

(III)

wherein $R^2$ represents an alkali metal and m denotes 0 or 1, which comprises:
reacting a penem compound of the following formula (I),

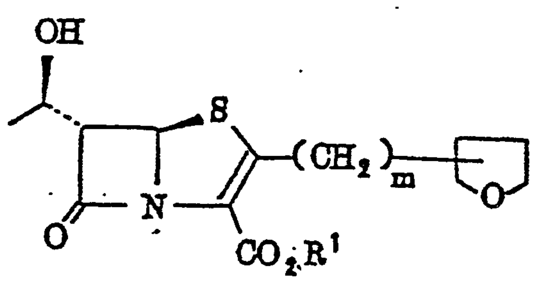

(I)

wherein $R^1$ is an allyl group which may have a substituent and m denotes 0 or 1, (a) with an alkali metal enolate of 1,3-diketone compound of formula (IIa),

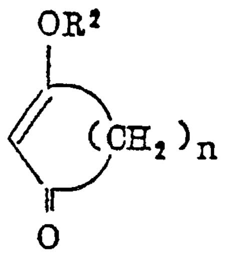

(IIa)

wherein $R^2$ has the same meaning as defined for formula (III), n denotes an integer of 2 to 4, and the hydrogen atoms on the ring may be substituted by a lower alkyl group, a lower alkoxycarbonyl group, a halogen atom, or an aryl group, in an organic solvent in the presence of a catalytic amount of a palladium complex, or (b) with a 1,3-diketone compound of formula (IIb),

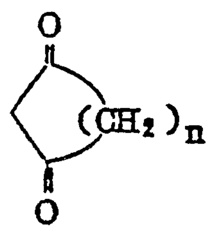

(IIb)

wherein n has the same meaning as defined above and the hydrogen atoms on the ring may be substituted by a lower alkyl group, a lower alkoxycarbonyl group, a halogen atom, or an aryl group, in an organic solvent in the presence of a salt of an alkali metal, and
adding water to the reaction mixture.

9. A process according to Claim 8, further comprising:
adding an inorganic base and water to said reaction mixture thus making the mixture alkaline,
separating the water layer from the organic layer and, optionally, concentrating the water layer,
adding a water soluble organic solvent to the water layer, and
separating the precipitated crystals of tne alkali metal salt hydrate of penem compound by filtration.

10. A process as claimed in Claim 9, wherein the inorganic base is added to the reaction mixture after water has been added thereto.

11. A process as claimed in any one of Claims 1 to 6, wherein the compound of formula (1) is reacted with a said compound of formula (IIb) in the presence of a catalytic amount of a palladium complex.

European Patent Office

# EUROPEAN SEARCH REPORT

Application Number

EP 90 30 8161

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 215 739 (CIBA-GEIGY)<br>* Page 36, example 3; page 40, example 6, claims * & JP-A-62 061 984 (Cat. D)<br>--- | 1-11 | C 07 D 499/88 |
| Y | EP-A-0 273 747 (SUNTORY LTD)<br>* Pages 21-22, examples 8-12; claims *<br>----- | 1-11 | |
| | | | **TECHNICAL FIELDS SEARCHED (Int. Cl.5)** |
| | | | C 07 D 499/00 |

The present search report has been drawn up for all claims

| Place of search | Date of completion of the search | Examiner |
|---|---|---|
| THE HAGUE | 11-09-1990 | CHOULY J. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document